# EUROPÄISCHE PATENTSCHRIFT

(11) **EP 0 557 786 B1**
(45) Veröffentlichungstag und Bekanntmachung des Hinweises auf die Patenterteilung: **08.05.1996**
(21) Anmeldenummer: 93101992.1
(22) Anmeldetag: 09.02.1993
(51) Int. Cl.: C07C 29/80, C07C 31/20

(54) **Verfahren zur Abtrennung von 1,4-Butandiol aus Hydriergemischen**
Method of separation of 1,4-butanediol from hydrogenation mixtures
Procédé de séparation de 1,4-butanediol à partir de mélanges d'hydrogénation

(30) Priorität: 22.02.1992 DE 4205471
(43) Veröffentlichungstag der Anmeldung: 01.09.1993
(73) Patentinhaber: BASF Aktiengesellschaft, D-67063 Ludwigshafen (DE)
(72) Erfinder: Gosch, Hans-Jürgen, Dr., W-6702 Bad Duerkheim (DE); Rust, Harald, W-6730 Neustadt (DE); Fischer, Rolf, Dr., W-6900 Heidelberg (DE); Hechler, Claus, Dr., W-6800 Mannheim 1 (DE); Pinkos, Rolf, Dr., W-6702 Bad Duerkheim (DE)

(56) Entgegenhaltungen:
- EP-A- 0 169 396
- WO-A-91/01960
- US-A- 4 032 583

## Beschreibung

Die Erfindung betrifft ein Verfahren zur Abtrennung von 1,4-Butandiol aus bei der katalytischen Hydrierung von Maleinsäure-, Fumarsäure- und/oder Bernsteinsäureestern gewonnenen Gemischen, die neben 1,4-Butandiol zusätzlich Tetrahydrofuran, Wasser, C₁-C₄-Alkohole, Bernsteinsäurediester, Hydroxibuttersäureester, Bernsteinsäure-alkylhydroxialkylester, gamma-Butyrolacton und Buttersäureester enthalten.

1,4-Butandiol, ein wichtiges Zwischenprodukt für die Herstellung von Polyestern, wird beispielsweise durch Hydrierung der Diethylester der Maleinsäure, Fumarsäure oder Bernsteinsäure hergestellt (EP 143 034). Bei derartigen Verfahren fallen Gemische an, die ganz überwiegend aus 1,4-Butandiol (BD) neben gamma-Butyrolacton (GBL), Tetrahydrofuran (THF), Bernsteinsäurediethylester, Ethanol, n-Butanol, Wasser und untergeordneten Mengen an hochsiedenden Nebenprodukten bestehen.

Für die Herstellung von BD-Lösungen ist es weiterhin möglich, Maleinsäureanhydrid in überschüssigen Alkoholen zu verwenden (EP 304 696). Bei der Hydrierung von Maleinsäureanhydrid, beispielsweise in n-Butanol, wird der zunächst entstehende Maleinsäuremonobutylester zu Gemischen umgesetzt, die ganz überwiegend aus BD, GBL, THF, Bernsteinsäuredibutylester (BSDE), n-Butanol, Wasser und geringen Mengen an Hochsiedern bestehen.

Es stellte sich daher die Aufgabe, das 1,4-Butandiol aus derartigen Hydriergemischen abzutrennen und die weiteren Wertprodukte GBL, THF und BSDE entweder ebenfalls in reiner Form zu gewinnen oder sie so abzutrennen, daß sie gegebenenfalls in die Hydrierstufe zurückgeführt werden können.

Als Alkohole in diesen Gemischen können C₁- bis C₄-Alkohole wie Methanol, Ethanol, n- und i-Propanol und/oder die vier isomeren Butanole enthalten sein. Dementsprechend können Ester aus C₄-Dicarbonsäuren und den genannten C₁-C₄-Alkoholen enthalten sein.

Besonders bevorzugt lassen sich Hydriergemische trennen, die n- oder i-Butanol und dementsprechend Carbonsäure-n- oder i-Butylester enthalten.

Obige Aufgabe wird erfindungsgemäß dadurch gelöst, daß
a) in einer ersten Kolonne mit einer praktischen Bodenzahl von 20 bis 70, wobei die Kolonne bei einem Kopfdruck von 50 bis 1100, insbesondere 50 bis 500 mbar und einer Kopftemperatur von 40 bis 120°C betrieben wird, Alkohol, Wasser und Tetrahydrofuran über Kopf abgetrennt werden,
b) das Sumpfprodukt der ersten Kolonne in eine zweite Kolonne, die eine praktische Bodenzahl von 30 bis 90 aufweist, eingespeist wird, und das bei einem Kopfdruck von 45 bis 250 mbar und einer Kopftemperatur von 45 bis 120°C erhaltene Kopfprodukt, das aus Alkohol und Buttersäureester besteht, ausgeschleust wird und in einem Seitenabzug gamma-Butyrolacton und Buttersäureester abgezogen werden und das Sumpfprodukt in einen Phasenscheider eingespeist wird,
c) die 1,4-Butandiol-reiche untere Phase des flüssigen zweiphasigen Gemisches des Phasenscheiders in eine dritte Kolonne eingespeist wird, die eine praktische Bodenzahl von 30 bis 90 aufweist, wobei bei einem Kopfdruck von 45 bis 250 mbar und einer Kopftemperatur von 45 bis 120°C Alkohol über Kopf abdestilliert wird, das 1,4-Butandiol/Bernsteinsäurediester-Azeotrop über einen Seitenstrom abgezogen wird und in den Phasenscheider zurückgeführt wird, und das 1,4-Butandiol zusammen mit Bernsteinsäure-alkyl-hydroxialkylester, Hydroxibuttersäurebutylester und Hochsiedern über den Sumpf abgeführt wird, und
d) die bernsteinsäurediesterreiche obere Phase im Phasenscheider abgetrennt wird.

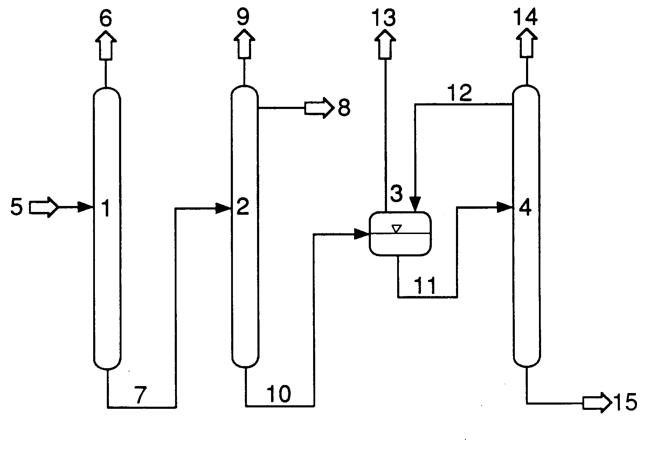

Das erfindungsgemäße Verfahren mit den wesentlichen Verfahrensmerkmalen ist in der Zeichnung - vereinfachtes Verfahrensschema - dargestellt und wird im folgenden näher beschrieben, wobei beispielhaft n-Butanol verwendet wird.

Der ersten Kolonne 1, mit einer praktischen Bodenzahl von 20 bis 70 wird ein Hydriergemisch 5 zugefahren, bestehend aus von Maleinsäure-, Fumarsäure- und/oder Bernsteinsäureestern gewonnenen Gemischen, die neben 1,4-Butandiol zusätzlich Tetrahydrofuran, Wasser, n-Butanol, Bernsteinsäurediester, Hydroxibuttersäureester, Bernsteinsäure-alkyl-hydroxialkylester, gamma-Butyrolacton und Buttersäureester enthalten.

Aus diesem Gemisch werden als Kopfprodukt 6 THF, Wasser und über 80 %, vorzugsweise 89 - 99 % des im Gemisch nicht chemisch gebundenen Butanols über Kopf abdestilliert. Die Temperaturen und Drücke am Kopf betragen beispielsweise 40 bis 100°C und 50 bis 500 mbar. Das Rücklaufverhältnis beträgt zwischen 0,1 und 0,8.

Das Kopfprodukt der ersten Kolonne, ein Gemisch aus THF, Wasser und Butanol, kann in an sich bekannter Weise zu reinem THF aufgearbeitet werden. Wasser wird ausgeschleust, Butanol in die Hydrierung zurückgefahren.

Der Sumpf 7 der ersten Kolonne wird etwa in die Mitte einer zweiten Kolonne 2, die eine praktische Bodenzahl von 30 bis 90 aufweist, eingespeist, in der über Kopf als Produktstrom 9 GBL, Buttersäurebutylester (BB) sowie vorwiegend bei der Destillation gebildetes n-Butanol abgetrennt werden. In einem Seitenabzug 8 werden Reste BB und über 80 % des im Gemisch enthaltenen GBL abgetrennt. Die Kopftemperatur beträgt dabei 45 bis 120°C bei 45 bis 250 mbar. Das Rücklaufverhältnis betragt z.B. 3 bis 15.

Auch das als Kopfprodukt der zweiten Kolonne anfallende n-Butanol kann, gegebenenfalls nach Abtrennung von Buttersäurebutylester, in die Hydrierstufe zurückgeführt werden.

Das im Seitenabzug der zweiten Kolonne gewonnene GBL kann gereinigt und beispielsweise für die Herstellung von N-Methylpyrrolidon genutzt oder aber in die Hydrierung zurückgeführt werden.

Das Sumpfprodukt 10 der zweiten Kolonne wird in einen Phasenabscheider 3 gefahren. Beim Abkühlen zerfällt das Sumpfprodukt in zwei flüssige Phasen. Die obere Phase 13 enthält vorwiegend BSDE, das abgetrennt wird und beispielsweise in die Hydrierstufe zurückgefahren werden kann. Die untere Phase 11, die vorwiegend aus BD, sowie geringeren Anteilen Hydroxybuttersäurebutylester (HOBB), BSDE und Bernsteinsäurebutylhydroxybutylester (B-BHOB) besteht, wird in eine dritte Kolonne 4 mit 30 bis 90 praktischen Böden eingespeist. Bei einer Kopftemperatur von 45 bis 120°C bei 45 bis 250 mbar wird neu gebildetes Butanol über Kopf als Produktstrom 14 abgetrennt. Über einen Seitenabzug 12 wird das BD/BSDE-Azeotrop zusammen mit restlichem und neu gebildetem GBL in den Phasenscheider zurückgefahren. Über den Sumpf 15 fließt BD, verunreinigt mit HOBB, B-BHOB und Hochsiedern ab. Das Rücklaufverhältnis beträgt z.B. 30 bis 50. Das so gewonnene BD kann in an sich bekannter Weise destillativ zu reinem BD verarbeitet werden.

Die für das neue Verfahren eingesetzten, BD enthaltenen Gemische fallen beispielsweise bei der katalytischen Hydrierung von Butylestern der Maleinsäure, Fumarsäure und/oder Bernsteinsäure an. Sie haben beispielsweise die folgende Zusammensetzung: 10 bis 70 Gew.-% Butanol, 0,5 bis 10 Gew.-% THF, 1,5 bis 9 Gew.-% Wasser, 2 bis 15 Gew.-% GBL, 20 bis 50 Gew.-% BD, 2 bis 15 Gew.-% BSDE, 0,1 bis 4 Gew.-% BB, 0,01 bis 3 Gew.-% HOBB, 0,01 bis 3 Gew.-% B-BHOB und 0,01 bis 5 Gew.-% Hochsieder.

Nach den Verfahren dieser Erfindung läßt sich 1,4-Butandiol aus Hydriergemischen besonders vorteilhaft abtrennen.

Bekannt war aus EP 169 396, daß Gemische aus BD und BSDE bei Temperaturen unterhalb von 108°C in zwei flüssige Phasen zerfallen: eine BD-reiche untere Phase und eine BSDE-reiche obere Phase. Es war jedoch nicht vorherzusehen, ob sich mit den weitere Komponenten enthaltenden BD/BSDE-Gemischen eine ausreichende, technisch nutzbare Phasentrennung erzielen lassen würde.

### Beispiel

Das zur Rektifiktion anstehende Hydriergemisch hatte folgende Zusammensetzung: 1,9 % THF, 58,9 % n-Butanol, 2,0 % GBL, 23,1 % BD, 0,4 % BB, 0,3 % HOBB, 6,4 % BSDE, 0,5 % B-BHOB, 0,5 % Hochsieder und 6,0 % Wasser.

Das Verfahren ist aus beigefügter Zeichnung ersichtlich. Über die Zuleitung (5) werden 100 Teile des Gemisches in die erste Kolonne (1) eingeleitet. Die Kolonne hat 35 theoretische Böden. Bei einer Kopftemperatur von 68°C und einem Kopfdruck von 115 mbar erhält man bei einem Rücklaufverhältnis von 0,5 66 Teile eines Kopfproduktes (6) mit der Zusammensetzung 2,9 % THF, 89,2 % Butanol und 7,9 % Wasser. Als Sumpfprodukt (7) werden 34 Teile eines Gemisches aus 1,4 % n-Butanol, 5,9 % GBL, 67,9 % BD, 1,2 % BB, 0,9 % HOBB, 1,5 % Hochsieder, 18,8 % BSDE, 1,5 % B-BHOB und 0,9 % weitere Verbindungen erhalten.

100 Teile Sumpfprodukt (7) aus der ersten Kolonne werden in die zweite Kolonne (2) mit 35 theoretischen Böden geleitet. Bei einer Kopftemperatur von 65°C, einem Kopfdruck von 100 mbar und einem Rücklaufverhältnis von 10 erhält man 15 Teile Kopfprodukt (9) der Zusammensetzung 82 % n-Butanol, 5,7 % GBL, 6,5 % BB und Wasser und 7 Teile Seitenstrom (8) aus dem Verstärkungsteil mit der Zusammensetzung 7 % n-Butanol, 77,4 % GBL, 9,3 % BD, 2,8 % BB und 3,5 % BSDE. Als Sumpfprodukt (10) werden 78 Teile eines Gemisches aus 0,2 % GBL, 71,7 % BD, 0,5 % HOBB; 9,3 % BSDE und 16,4 Teile B-BHOB und 1,9 % Hochsieder erhalten.

Das Sumpfprodukt (10) wird in den Phasenscheider (3) geleitet.

100 Teile untere Phase (11) des Phasenscheiders werden in die dritte Kolonne (4) mit 35 theoretischen Böden geleitet. Bei einer Kopftemperatur von 46°C, einem Kopfdruck von 100 mbar und einem Rücklaufverhältnis von 40 erhält man 4 Teile Kopfprodukt (14) der Zusammensetzung 76 % n-Butanol und 24 % Wasser, 16 Teile Seitenstrom (12) aus dem Verstärkungsteil mit der Zusammensetzung 2 % n-Butanol, 2 % GBL, 61 % BD, 33 % BSDE und 2 % andere. Als Sumpfprodukt (15) werden 80 Teile eines Gemisches aus 90,5 % BD, 5,5 % B-BHOB und 4 % Hochsieder erhalten.

Der Seitenstrom (12) wird in den Phasenscheider (3) geleitet.

Sumpfprodukt (10) und Seitenstrom (12) werden im Phasenscheider (3) vermischt. Ein Phasenzerfall ermöglicht Abzug der in der Hauptsache aus BSDE bestehenden oberen Phase (13), die in die Hydrierstufe zurückgegeben wird. Die untere Phase (11) die aus BD, HOBB, BSDE, Hochsieder und B-BHOB besteht, wird in die dritte Kolonne geleitet.

## Patentansprüche

1. Verfahren zur Abtrennung von 1,4-Butandiol aus bei der katalytischen Hydrierung von Maleinsäure-, Fumarsäure- und/oder Bernsteinsäureestern gewonnenen Gemischen, die neben 1,4-Butandiol zusätzlich Tetrahydrofuran, Wasser, C₁-C₄-Alkohole, Bernsteinsäurediester, Hydroxibuttersäureester, Bernsteinsäure-alkyl-hydroxialkylester, gamma-Butyrolacton und Buttersäureester enthalten,
dadurch gekennzeichnet, daß
a) in einer ersten Kolonne mit einer praktischen Bodenzahl von 20 bis 70, wobei die Kolonne bei einem Kopfdruck von 50 bis 1100, insbesondere 50 bis 500 mbar und einer Kopftemperatur von 40 bis 120°C betrieben wird, Alkohol, Wasser und Tetrahydrofuran über Kopf abgetrennt werden,
b) das Sumpfprodukt der ersten Kolonne in eine zweite Kolonne, die eine praktische Bodenzahl von 30 bis 90 aufweist, eingespeist wird, und das bei einem Kopfdruck von 45 bis 250 mbar und einer Kopftemperatur von 45 bis 120°C erhaltene Kopfprodukt, das aus Alkohol und Buttersäureester besteht, ausgeschleust wird und in einem Seitenabzug gamma-Butyrolacton und Buttersäureester abgezogen werden und das Sumpfprodukt in einen Phasenscheider eingespeist wird,
c) die 1,4-Butandiol-reiche untere Phase des flüssigen zweiphasigen Gemisches des Phasenscheiders in eine dritte Kolonne eingespeist wird, die eine praktische Bodenzahl von 30 bis 90 aufweist, wobei bei einem Kopfdruck von 45 bis 250 mbar und einer Kopftemperatur von 45 bis 120°C Alkohol über Kopf abdestilliert wird, das 1,4-Butandiol/Bernsteinsäurediester-Azeotrop über einen Seitenstrom abgezogen wird und in den Phasenscheider zurückgeführt wird, und das 1,4-Butandiol zusammen mit Bernsteinsäure-alkyl-hydroxialkylester, Hydroxibuttersäurebutylester und Hochsiedern über den Sumpf abgeführt wird, und
d) die bernsteinsäurediesterreiche obere Phase im Phasenscheider abgetrennt wird.

2. Verfahren nach Anspruch 1, dadurch gekennzeichnet, daß die Carbonsäureester als Alkoholkomponente C₁- bis C₄-Alkohole enthalten.

## Claims

1. A process for the separation of 1,4-butanediol from mixtures obtained from the catalytic hydrogenation of eaters of maleic acid, fumaric acid and/or succinic acid, which mixtures, in addition to 1,4-butanediol, additionally contain tetrahydrofuran, water, C₁-C₄-alcohols, diesters of succinic acid, esters of hydroxybutyric acid, alkyl hydroxyalkyl eaters of succinic acid, gamma-butyrolactone and esters of butyric acid, which comprises
a) removing alcohol, water and tetrahydrofuran at the top of a first column having 20 to 70 actual plates, the column being operated at a head pressure of from 50 to 1100 mbar, in particular from 50 to 500 mbar, and a head temperature of from 40 to 120°C,
b) feeding the bottom product from the first column to a second column which has from 30 to 90 actual plates, removing the head product comprising alcohol and esters of butyric acid which is obtained at a head pressure of from 45 to 250 mbar and a head temperature of from 45 to 120°C, removing gamma-butyrolactone and esters of butyric acid via a side outlet, and feeding the bottom product into a phase separator,
c) feeding the 1,4-butanediol-rich lower phase of the liquid two-phase mixture from the phase separator into a third column which has from 30 to 90 actual plates, in which alcohol is distilled off at the top at a head pressure of from 45 to 250 mbar and a head temperature of from 45 to 120°C, and in which the 1,4-butanediol/succinic acid diester azeotrope is removed via a side stream and fed back into the phase separator, and the 1,4-butanediol is removed at the bottom together with alkyl hydroxyalkyl esters of succinic acid, butyl hydroxybutyrate and high-boiling components, and
d) removing the succinic acid diester-rich upper phase in the phase separator.

2. A process as claimed in claim 1, wherein the carboxylic acid esters contain C₁ to C₄-alcohols as alcohol component.

## Revendications

1. Procédé de séparation du 1,4-butanediol d'avec des mélanges obtenus au cours de l'hydrogénation catalytique d'esters de l'acide maléique, de l'acide fumarique et/ou de l'acide succinique, qui, outre du 1,4-butanediol, contiennent complémentairement du tétrahydrofuranne, de l'eau, des alcools en C₁ à C₄, des diesters de l'acide succinique, des esters de l'acide hydroxybutyrique, des esters alkyl-hydroxyalkyliques de l'acide succinique, de la gamma-butyrolactone et des esters de l'acide butyrique, caractérisé en ce que
a) dans une première colonne comportant un nombre de plateaux pratique de 20 à 70, où la colonne travaille à une pression de tête de 50 à 1100 mbars, plus particulièrement 50 à 500 mbars et à une température de tête de 40 à 120°C, on sépare l'alcool, l'eau et le tétrahydrofuranne en tête,
b) on injecte le produit de fond de la première colonne dans une seconde colonne qui comporte un nombre de plateaux pratique de 30 à 90 et on expulse le produit de tête obtenu à une pression de tête de 45 à 250 bars et une température de tête de 45 à 120°C, qui se compose d'alcool et d'ester de l'acide butyrique, et, par un dispositif de prélèvement latéral, on prélève de la gamma-butyrolactone et de l'ester d'acide butyrique et on injecte le produit de fond dans un séparateur de phases,
c) on injecte la phase inférieure riche en 1,4-butanediol du mélange à deux phases liquide du séparateur de phases dans une troisième colonne qui présente un nombre de plateaux pratique de 30 à 90, où à une pression de tête de 45 à 250 et une température de tête de 45 à 120°C, on évacue l'alcool en tête par distillation, on prélève l'azéotrope 1,4-butanediol/diester de l'acide succinique par l'intermédiaire d'un courant latéral et on le ramène au séparateur de phases et on évacue le 1,4-butanediol en même temps que l'ester alkyl-hydroxyalkylique de l'acide succinique, l'ester butylique de l'acide hydroxybutyrique et des composés à points d'ébullition supérieurs par le fond et
d) on sépare la phase supérieure riche en diester de l'acide succinique dans le séparateur de phases.

2. Procédé suivant la revendication 1, caractérisé en ce que les esters d'acides carboxyliques contiennent des alcools en C₁ à C₄ à titre de composants alcool.
